# EUROPEAN PATENT SPECIFICATION

(11) **EP 0 672 453 B1**
(45) Date of publication and mention of the grant of the patent: **02.11.2005**
(21) Application number: 95301022.0
(22) Date of filing: 17.02.1995
(51) Int. Cl.: B01J 32/00, B01J 35/10, B01J 37/00, C07C 67/05, C07C 69/15, B01J 23/56

(54) **Process for the preparation of fluid bed vinyl acetate catalyst**
Verfahren zur Herstellung eines Katalysators für die Herstellung von Vinylacetat in einem Wirbelbett
Procédé de préparation d'un catalyseur pour préparer l'acétate de vinyle dans un lit fluidisé

(30) Priority: 22.02.1994 US 200130; 20.01.1995 US 376180
(43) Date of publication of application: 20.09.1995
(60) Divisional application: 99112752.3
(73) Proprietor: THE STANDARD OIL COMPANY, Cleveland, Ohio 44114-2375 (US)
(72) Inventor: Blum, Patricia Rae, Macedonia, Ohio 44056 (US); Cirjak, Larry Michael, Burton, Ohio 44021 (US); Lemanski, Michael Francis, Chester, New York 10918 (US); Paparizos, Christos, Willowick, Ohio 44095 (US); Pepera, Marc Anthony, Northfield, Ohio 44067 (US); Suresh, Devasirvatham Dhanarah, Hudson, Ohio 44236 (US)
(74) Representative: Perkins, Nicholas David

(56) References cited:
- EP-A- 0 032 298
- EP-A- 0 082 222
- EP-A- 0 283 649
- EP-A- 0 431 478
- FR-A- 2 010 775
- FR-A- 2 276 285
- GB-A- 2 003 878
- US-A- 1 266 623
- US-A- 3 822 308
- US-A- 4 631 264
- US-A- 5 179 056

## Description

### BACKGROUND OF THE INVENTION

### Field of the Invention

The present invention relates to a process for producing a fluid bed palladium-promoted catalyst useful in the production of vinyl acetate from ethylene, acetic acid and oxygen-containing gas. In addition, the present invention relates to a novel fluid bed support and process of using the support for the manufacture of palladium-promoted fluid bed catalyst used in the manufacture of vinyl acetate.

The production of vinyl acetate by reacting ethylene, acetic acid and oxygen together in the gas phase in the presence of a catalyst is known. Typically, the catalysts are in fixed bed form and supported on a porous carrier material such as silica or alumina.

Early examples of these catalysts show that palladium and gold are distributed more or less uniformly throughout the carrier (see, for example, U.S. Patent Nos. 3,275,680, 3,743,607 and 3,950,400 and Great Britain Patent No. 1,333,449 and South African Patent No. 687,990). Subsequently, it was recognized that this was a disadvantage since it was found that the material on the inner part of the carrier did not contribute to the reaction since the reactants did not significantly diffuse into the carrier. To overcome this problem, new methods of catalyst manufacture were devised with the aim of producing catalyst in which the active components were concentrated on the outer-most shell of the support. For example, Great Britain Patent No. 1,500,167 claims catalyst in which at least ninety percent of the palladium and gold is distributed in that part of the carrier particle which is not more than thirty percent of the particle radius from the surface. In addition, Great Britain Patent No. 1,283,737 teaches that the degree of penetration into the porous carrier can be controlled by pre-treating the porous carrier with an alkaline solution of, for example, sodium carbonate or sodium hydroxide. Another approach which has been found to produce particularly active catalyst is described in U.S. Patent No. 4,048,096 and other methods of producing shell-impregnated catalyst are disclosed in U.S. Patent Nos. 4,087,622 and 5,185,308. Each of these patents is primarily concerned with the manufacture of fixed bed catalyst useful for the manufacture of vinyl acetate. However, U.S. Patent No. 3,950,400 also discloses that the catalyst disclosed therein may be used in a fluid bed reactor. In addition, Great Britain Patent No. 1,266,623 allegedly discloses a fluid bed catalyst for vinyl acetate manufacture which comprises palladium promoted with various alkali, alkaline earth or other metals.

It would be economically beneficial if the manufacture of vinyl acetate could be performed in a fluid bed process as well as a fixed bed process. Some of the typical benefits from a fluid bed process would be that the fluid bed reactor design is simpler than a multi-tubular fixed bed reactor, increased catalyst life is to be expected because no deactivation would take place due to hot spots which are typical of a fixed bed reactor, continuous addition of make-up catalyst can maintain peak performance and virtually eliminate catalyst change-outs, and higher production rates can be expected because substantially higher oxygen levels may be safely fed into the reactor without producing a flammable mixture.

Until the discovery of the process of the present invention, the preparation of palladium-promoted catalyst in fluid bed form has not led to catalyst having the necessary properties leading to a viable economical fluid bed process for the manufacture of vinyl acetate. The process of the present invention overcomes the problems associated with the prior art resulting in a catalyst giving high performance and adequate attrition resistance so that it may be used in the manufacture of vinyl acetate.

### Summary of the Invention

It is the primary object of the present invention to provide a process for the manufacture of a fluid bed palladium-metal-promoted alkali metal catalyst useful in the manufacture of vinyl acetate.

Additional objects and advantages of the invention will be set forth in part in the description which follows and, in part, will be obvious from the description or may be learned by practice of the invention. The objects and advantages of the invention may be realized and attained by means of the instrumentalities and combinations particularly pointed out in the appended claims.

To achieve the foregoing objects of the present invention, the process of manufacturing a fluid bed vinyl acetate catalyst characterized by the following formula comprising Pd-M-A wherein M equals barium, gold, lanthanum, niobium, cerium, zinc, lead, calcium, strontium, antimony, or mixtures thereof; and A equals at least one alkali metal or mixture thereof comprises impregnating a pre-formed microspheroidal support wherein at least 50% of the particles have a particle size selected to be below 10⁻⁴ m (100 microns) with a solution comprising a halide-free metal salt of the palladium, M and at least one alkali metal and drying the impregnated catalyst. The substantially inert particulate support typically comprises microspheroidal particles selected from the group consisting of alumina, silica, zirconia, or mixtures thereof.

In another embodiment of the present invention the process is performed using an aqueous solution free or substantially free of any organic solvent.

In a preferred embodiment of the present invention the metal salt of the alkali metal is separately impregnated onto the support, preferably subsequent to the impregnation of the solution comprising the salts of palladium and M element onto the support material.

In another embodiment of the present invention, the impregnated support is heated under reducing conditions to form a deposit of palladium and M on the surface of the support. The reduction can take place either before or after the deposition of the alkali metal solution.

In a still further preferred embodiment of the present invention the catalyst is dried at a temperature below 80°C, preferably between about 60° to 70°C.

In another preferred embodiment of the present invention the particle size(particle diameter) of the substantially inert support material is selected such that at least 50% of the particles are below about 6 x 10⁻⁵ m (60 microns). Preferably, at least 75% of the particles are below 10⁻⁴ m (100 microns), especially preferred being at least 85% below 10⁻⁴ m (100 microns). Finally the preferred support is substantially free of sodium.

The support for the manufacture of the vinyl acetate catalyst comprises a mixture of substantially inert microspheroidal particles having a pore volume of between 0.2 to 0.7cc/g, a surface area of between 100 to 200 m²/g and at least 50% of said particle are less than 10⁻⁴ m (100 microns).

In a preferred aspect of this embodiment of the present invention, at least 75% of the particles are below 100 microns, especially preferred being at least 85% below 100 microns.

In another embodiment of the present invention, the support for the manufacture of the vinyl acetate catalyst comprises microspheroidal inert particles, preferably silica, zirconia, alumina or mixtures thereof wherein said particles have a pore volume of between 0.2 to 0.7 cc/g, a surface area of between 100 to 200 m²/g and are obtained from a mixture of 80 to 20% inert support sol and 20 to 80% dried inert particles.

In a preferred embodiment of this aspect of the present invention, the pore volume of the inert particles is between 0.3 to 0.65 cc/g, especially preferred being 0.4 cc to 0.55 cc/g.

In a further preferred embodiment of this aspect of the present invention, the surface area is between 110 to 195 m²/g, especially preferred being 120 to 195 m²/g.

In a further aspect of this embodiment of the present invention, the silica microspheroidal support material is manufactured by mixing between 20% to 80% silica sol with 80% to 20% silica aerosil, spray drying said mixture at an elevated temperature of between 125°C to 280°C, preferably 130° to 240°C, and calcining said spray dried particles preferably at a temperature of between 550° to 700°C, preferably between 630° to 660°C to form the support material.

The substantially inert support for a fluid bed vinyl acetate catalyst may be manufactured by a process comprising mixing 80 to 20 wt% of an aqueous sol comprising substantially inert microspheroidal particles with 20 to 80 wt% of solid substantially inert particulate material to form an aqueous mixture, spray drying said aqueous mixture, and calcining said particles to form said substantially inert support.

Typically, the weight percent of the palladium, M and alkaline in the catalyst of the present invention are: 0.1 to 5.0 wt% palladium, preferably 0.2 to 4.0 wt%, most preferably 0.3 to about 1.0 wt%; greater than 0 to 10 wt% alkali metal, preferably 0.1 to 8.0 wt%, most preferably 0.1 to 5.0 wt%; greater than 0 to about 5.0 wt% M, preferably 0.1 to about 4.0 wt%, most preferably 0.1 to 3.0 wt%.

### Detailed Description of the Invention

Reference will now be made in detail to the present preferred embodiment of the invention of which the following examples are set forth for illustrative purposes only.

### Reactor Testing

The catalysts were tested in a bench scale fluid bed reactor with a maximum catalyst capacity of 40 cc. Thirty cc of catalyst or catalyst-plus-diluent was the typical volume of solid loaded into the reactor. In general, sufficient catalyst was used such that the reactor contained 0.093 g of palladium metal with each catalyst evaluation. A total of 30 cc volume was obtained by mixing sufficient inert microspheroidal silica with the active catalyst prior to reactor testing. The reactor was equipped with two feed inlets. For some of the experiments of this study, ethylene, acetic acid, and oxygen all entered the reactor through the lower inlet and nitrogen only was fed through the central inlet. In other tests, additional oxygen was fed through the central feed inlet. This central inlet was located 6.35 cm (2.5") above the lower feed inlet.

The reactor pressure was controlled at 7.93 bar (115 psig) and all lines leading to and from the reactor were heat traced and maintained at 150-155°C order to prevent condensation of liquid feeds or products. Typical temperatures for the fluid bed reactor can vary from 100° to 250°C, preferably 135° to 190°C.

The gaseous reactor effluent was analyzed on-line using a Hewlett Packard Model 5890 gas chromatograph equipped with both TCD and FID detectors. Oxygen, nitrogen, ethylene and carbon dioxide were separated on a 13x mole sieve column parallel with 10% carbowax 20M on 80/100 Chromosorb WAW and 23% SP2700 on 80/100 Chromosorb PAW, and quantitated with the TCD. Vinyl acetate and acetic acid were separated on a 4% carbowax 20M on 80/120 carbopack column and quantitated with the FID.

### Support Preparation

Two types of preformed microspheroidal silica were prepared and utilized as supports in the practice of the present invention. Prior to use, all supports were sieved and a specific particle size distribution of the support was used in all catalyst preparations:

| | |
|---|---|
| 5% | of the particles are less than 1.05 x 10⁻⁴ m (105 microns) but greater than 8.8 x 10⁻⁵ m (88 microns). |
| 70% | of the particles are less than 8.8 x 10⁻⁵ m (88 microns) but greater than 4.4 x 10⁻⁵ m (44 microns) |
| 25% | of the particles are less than 4.4 x 10⁻⁵ m (44 microns) |

It should be understood the particle size distribution recited above is not intended to be limiting and that variations in this distribution are contemplated depending upon reactor size and operating conditions.

### Support 1

Support 1 was prepared by spray drying a mixture of Nalco (Nalco Chemical Company) silica sol 1060 and DeGussa Aerosil® (DeGussa Chemical Company) silica. In the dried support, 80% of the silica came from the sol and 20% of the silica came from the Aerosil. The spray dried microspheres were calcined in air at 640°C for 4 hours.

Aerosil® silica is the trade name of Degussa's fumed silica. This material has high surface area (~200 m²/g), essentially no micropores, uniform particle size distribution in the nm-range (1 x 10⁻⁹ meter), and is free of sodium. Fumed silica having properties comparable to Aerosil® may be produced by other companies and may be used in the place of Aerosil® in the preparation of Support 1.

Nalco silica sol 1060 is particularly advantageous for use in our application because of large mean particle size of the silica particles in the sol, 60 millimicrons. These larger silica particles pack less efficiently than smaller sol particles (~30 millimicrons as in Nalco 2327) and yield a final support higher in pore volume in the mesopore region and lower in micropore volume. Other silica sols which have a similarly large (~40-80millimicron) mean particle size of the silica may be utilized in the place of the 1060 silica sol in the preparation of Support 1.

### Support 2

A series of microspheroidal supports (Supports 2A-2D) containing KA-160 (Sud Chemie) were prepared as follows:

### Support 2A: 75% SiO₂ from KA-160 with 25% SiO₂ from Sol

750 g of KA-160 was ground to pass through a 35 mesh screen and washed to remove any soluble impurities, such as chloride ions. This solid silica was then mixed with 694.4 g of Snotex-N-30 (Nissan Chemical) (36 wt% solids) silica sol and 556 g distilled water. This mixture was milled overnight in a jar mill. The smooth slurry was then spray dried to form microspheroidal particles suitable for use in a fluid bed reactor. The microspheroidal support was then calcined at 640°C in air for 4 hours.

The role of the KA-160 support is to provide much of the pore structure within the microspheroidal particle. The fixed bed support, KA-160, is produced by Sud Chemie and has properties which are advantageous for use in vinyl acetate catalyst preparation. Moderate surface area (160 m²/g), little or no microporosity, and substantial porosity (~ 0.57 cc/g) in the mesopore region are advantageous properties of KA-160. Alternative fixed bed catalyst supports are available with surface area and pore volume properties similar to KA-160 (little or no micropores, mesopore volume of ~1.5-0.25 cc/g, and surface area 80-200 m²/g). These supports may be utilized in the place of KA-160 in the preparation of Support 2.

### Support 2B: 65% SiO₂ from KA-160 with 35% SiO₂ from Sol

This support was prepared in the same manner as Support 2A except that 227.5 g of KA-160, 408.3 g of Snotex-N-30 (30 wt% solids) and 64 g of distilled water were used.

### Support 2C: 50% SiO₂ from KA-160 with 50% SiO₂ from Sol

This support was prepared in the same manner as Support 2A except that 175 g of KA-160 and 583.3 g of Snotex-N-30 (30 wt% solids) were used.

### Support 2D: 75% SiO₂ from KA-160 with 25% SiO₂ from Sol

This support was prepared in the same manner as Support 2A except that 262 g of KA-160, 219 g of Nalco 2327 (40 wt% solids) (Nalco Chemicals Company) and 219 g of distilled water were used.

Each type of microspheroidal silicas prepared above may be used advantageously in the preparation of fluid bed vinyl acetate monomer catalyst according to the process of the present invention. For use in the manufacture of fluid bed catalysts via impregnation with active metals, these supports provided unexpected superior physical properties for the vinyl acetate catalysts of the present invention compared to any readily available supports. Selected analytical data on all supports are included in Table 1 below.

**TABLE 1**

| PHYSICAL PROPERTIES OF CUSTOMIZED MICROSPHEROIDAL SILICA SUPPORTS | | | | | | | |
|---|---|---|---|---|---|---|---|
| Support | Wt% Solids in Slurry | Pore Vol r≤4,500A (cc/g) | Tot Pore Vol (cc/g) | Ap Bulk Density (g/cc) | SA m²/g | Calcin Time/Temp | Attrition Resist Loss 0-20 hrs |
| Support 1 | 62 | 0.39 | 0.46 | 0.78 | 124.4 | 4 hr/640°C | <5% |
| Support 2A | 50 | 0.60 | 0.60 | 0.65 | 175.5 | 4 hr/640°C | 0.33% |
| Support 2B | 50 | 0.39 | 0.39 | 0.72 | 184.4 | 4 hr/640°C | 0.35% |
| Support 2C | 46 | 0.27 | 0.33 | 0.77 | 191.9 | 4 hr/640°C | 1.65% |
| Support 2D | 50 | 0.62 | 0.63 | 0.60 | 156.0 | 4 hr/640°C | |

### Catalyst Preparation

The general method utilized in the preparation is summarized below.

Typically, the microspheroidal support is impregnated with a solution (or solutions) of the active metals using the incipient wetness technique. Halide free compounds of the active metals, palladium, M element (e.g.gold) and potassium acetate, may be dissolved in the appropriate ratios in a suitable solvent, then impregnated upon the microspheroidal support. In general, it is desirable if all of the active metals to be used in a catalyst preparation are dissolved in a single portion of solvent which is of the volume just adequate to fill the pore volume of the support. In some instances a desired promoter may not be soluble in the same solvent as the other metal compounds to be used. In this case a solution containing some of the metal components may be impregnated upon the support, followed by impregnating a second solution containing the remaining components. Solvents which are useful include water and volatile organic solvents such as: carboxylic acids with four carbons or less, alcohols, ethers, esters, and aromatics. After the wet catalyst is dried, it may be used for the production of vinyl acetate or it may first be reduced by means known to those skilled in the art.

In general, when acetic acid is present and the catalyst is heated at an elevated temperature (~100°C) the catalyst darkens to black and becomes inactive. Additionally, when a solution of palladium acetate (with or without other metal acetates) is heated to too high a temperature or for too long, the solution changes color from the original red-orange to a greenish color and a black precipitate forms. In general, 60°C is a safe temperature to work at, but up to ~80°C has been used for brief periods of time, to dissolve the palladium acetate.

### Example 1

A catalyst having the following composition 0.75 wt% Pd, 0.32 wt% Au and 2.88 wt% K was prepared by dissolving palladium acetate in an acetic acid solution of the gold acetate reagent described in U.S. Patent 4,933,204 and impregnating this combined solution upon a preformed microspheroidal Support 2A identified above. The solid was dried at 60°C using a rotary evaporator (rotovap), then the Pd and Au were reduced with an aqueous solution of hydrazine (no alkali hydroxide). The solid was washed to remove hydrazine, dried and potassium acetate was impregnated upon the solid. A 12.67g(16.7cc) charge of catalyst was placed in the reactor for testing. The results of reactor testing of this catalyst at various conditions are set forth below in Table 2. These results show an 18.2% conversion with 83% selectivity using 10.55 O₂, 14.31% HOAc, at 164.9°C.

### Example 2

The catalyst of this example had a composition of 1.07 wt% Pd, 0.40 wt% Au and 2.89 wt% K and was prepared according to the procedure set forth in Great Britain Patent 1,266,623 except that the support was the same as used in Example 1. A 8.68g(11.3cc) charge of catalyst was placed in the reactor for testing. The results of testing of this catalyst at various conditions is set forth below in Table 2 and gave 8.1% ethylene conversion and 84.4% vinyl acetate selectivity using 7% O₂, 10% HOAc, at 159°C.

### Example 3

The procedure of Example 2 was repeated to produce a catalyst having a composition as follows: 1.01 wt% Pd, 0.38 wt% Au and 2.60 wt% K. However, Support 1 identified above was utilized. A 9.2g(10.6cc) charge of catalyst was placed in the reactor for testing. The reactor testing at various conditions is set forth below in Table 2. The catalyst gave C₂H₄ conversion of 8.6 and VA selectivity of 85.3 under the same conditions as set forth in Example 2.

The performance of the catalyst of Examples 2 and 3 is very similar but the catalyst prepared on the Support 1 appears to be slightly more active. As the compositions of these two catalysts are nearly identical, the difference in activity may be due to the different supports.

### Example 4

This catalyst was prepared according to the teachings of U.S. Patent 3,950,400 except that microspheroidal (fluid bed) Support 1 as described above was utilized. The composition was 0.82 wt% Pd, 0.40 wt% Au, 0.13 wt% Ba, 2.69 wt% K. The acetic acid was carefully removed under vacuum (using a rotovap) at 60°C. This solid remained tan in color. A 11.57g(13.4cc) charge of catalyst was placed in the reactor for testing. Reactor testing of this catalyst set forth in Table 2 demonstrated it to be highly active and selective. At 164°C using 7% oxygen and 14% acetic acid, 12.5% ethylene conversion was obtained with 87.2% selectivity.

### Example 5

A catalyst having the following composition: Pd 0.81 wt%, 0.34 wt% Au and 2.71 wt% K was prepared by dissolving palladium acetate (PdAc) and potassium acetate (KAc) in acetic acid, then adding gold acetate and impregnating it on Support 1. The acetic acid was removed under vacuum, at 60°C. This solid was tan in color at this point. The preparation of this catalyst is similar to that of Example 1 except there was no catalyst reduction prior to testing. A 11.75g(13.2cc) charge of catalyst was placed in the reactor for testing. The results of testing this catalyst under various conditions is set forth in Table 2. The catalyst gave 9.2% conversion with 87.8% VA selectivity.

### Example 6

A catalyst having the following composition: 0.77 wt% Pd, 0.40 wt% Au and 2.2 wt% K was prepared as with Example 5. The solid was then subjected to a hydrazine reduction, washed with water to remove hydrazine, and additional potassium acetate was added. A 14.25g(17.6cc) charge of catalyst was placed in the reactor for testing. Excellent reactor results were obtained as shown in Table 2. This catalyst gave similar results, 10.17% conversion with 85.7% selectivity, as compared with Example 5.

A variety of Pd/M/K on silica-type catalysts were prepared wherein M is not gold. Metals evaluated included M =Ba, La, Sb, Pb, Ce, Nb, Ca, Zn, and Sr. The following examples are illustrative of these various metals.

### Example 7

The catalyst was prepared with the lower level of palladium which is typically used with Bayer-type catalysts, 0.88 wt% Pd, but which is typically too inactive for use with Hoechst-type catalysts along with 0.88 wt% Ba. Acetic acid was the solvent. The catalyst had 2.9 wt% K. A 15.52g(21.0cc) charge of catalyst was placed in the reactor for testing. The results of testing conversions approaching 10% ethylene with 81% selectivity to VA were obtained under various conditions as set forth below in Table 2. The catalyst suffered some deactivation by exposure to an elevated temperature (100°C) while acetic acid was still present.

### Example 8

A catalyst having 0.41 wt% Pd, 0.49 wt% Ba and 2.2 wt% K was prepared using water as the sole solvent. The mixture of palladium acetate, potassium acetate and barium acetate is sufficiently soluble in distilled water that water can be used as the sole solvent. A 24.77g(30.0cc) charge of catalyst was placed in the reactor for testing. Reactor testing of this catalyst under various conditions is set forth below in Table 2 and gave 10% ethylene conversion at 85% selectivity to VAM.

The use of water as the impregnating solvent instead of acetic acid has several significant advantages. Water is certainly less expensive, less toxic and less corrosive than acetic acid. All of which will give a less expensive process using water. Additionally, water does not act as a reducing agent for the palladium. When heated at 100°C in the oven, the catalyst prepared with acetic acid darkened to near black, whereas the analogous catalyst prepared in water, retained its tan color and still retained its excellent reactor performance. Finally, water would be a more benign solvent with respect to any detrimental effects upon the support.

### Example 9

A solution of palladium acetate, potassium acetate and antimony acetate in acetic acid were impregnated upon the preformed microspheroidal support. The wet solid was dried at 60°C under vacuum. No pre-reduction of the catalyst was performed. The resulting catalyst comprised 0.81 wt% Pd, 0.70 wt% Sb and 2.9 wt% K. A 10.95g(12.8cc) charge of catalyst was placed in the reactor for testing. Reactor testing shown in Table 2 gave ethylene conversions of nearly 17% with 89% selectivity at only 9 mole% oxygen in the feed mixture.

### Example 10

The addition of barium to an antimony containing catalyst substantially reduced catalyst activity. The catalyst tested had a composition (wt%) of 0.71 Pd, 0.71 Ba, 0.71 Sb and 2.6 K. A 10.95g(13.5cc) charge of catalyst was placed in the reactor for testing. There is no synergy between the antimony and the barium at the levels evaluated as shown by the results in Table 2 below.

### Examples 11 and 12

A mixture of palladium acetate, lanthanum acetate and potassium acetate was quite soluble in acetic acid. Support 1 was used for Example 11 and Support 2A for Example 12. This solution impregnated upon the preformed support and dried under vacuum resulted in an excellent catalyst as shown in Table 2 below. The composition of catalysts 11 and 12, respectively, in weight percent were as follows: 0.77 Pd, 0.70 La, 2.7 K; 0.80 Pd, 0.57 La, 3.1 K. For example 11 a 10.95g(13.0cc) charge of catalyst was placed in the reactor for testing. For example 12 a 10.95g(15.0cc) charge of catalyst was placed in the reactor for testing. Conversions and selectivities were slightly lower than with the antimony-containing catalyst, but were still very good.

### Example 13

The mixture of palladium acetate, lanthanum acetate and potassium acetate was dissolved in water instead of acetic acid resulting in a catalyst having the following composition: 0.15 wt% Pd, 0.34 wt% La, 1.4 wt% K. A 25.2g (30.0cc) charge of catalyst was placed in the reactor for testing. Considering the low level of palladium present, the ethylene conversion of 8% as shown in Table 2 was quite good.

### Example 14

Niobium oxalate, the source of niobium utilized, was insoluble in acetic acid. For that reason the niobium oxalate was pre-impregnated onto Support 1 using an aqueous solution. After drying the support, an acetic acid solution of palladium acetate and potassium acetate was impregnated upon the support. A 11.04g(14.0cc) charge of catalyst was placed in the reactor for testing. Resulting catalyst composition was 0.81 wt% Pd, 0.64 wt% Nb, 3.1 wt% K. Reactor performance was adequate at -9% conversion and 84% selectivity, but this catalyst appeared to deactivate more rapidly than expected.

### Examples 15 and 16

Calcium was added as the promoter at two different levels: (1) the same mole % as barium in Example 7, and (2) at near the wt% level as barium in Example 7. In each case, Support 2A was used. For example 15 a 10.95g(15.8cc) charge of catalyst was placed in the reactor for testing. For example 16, a 10.95g(15.4cc) charge of catalyst was placed in the reactor for testing. Neither catalyst performed well as shown in Table 2, but the lower level of calcium gave higher conversions and higher selectivities. It is possible that adjusting the calcium level further could improve catalyst performance.

### Examples 17 and 18

Cerium promoted catalyst (Example 17) and zinc promoted catalyst (Example 18) were prepared as described in the general procedure set forth above with the metals being dissolved in acetic acid and drying at 60°C under vacuum. In each case, Support 2A was utilized. The final composition of the catalyst were: Example 17--0.80 wt% Pd, 0.69 wt% Ce, 2.8 wt% K; Example 18--0.81 wt% Pd, 0.33 wt% Zn and 2.9 wt% K. A 10.96g(15.6cc) charge of catalyst was placed in the reactor for testing for example 17. For example 18, a 10.96g(15.6cc) charge was used. Tests of these catalysts showed potential as shown in Table 2. Optimization of promoter level and reduction treatment could be beneficial. In particular, cerium showed very good initial activity.

### Examples 19 and 20

The catalyst of Examples 19 and 20 were prepared on the same support and utilizing substantially the same procedure set forth in Examples 17 and 18 above except that Pb and Sr were substituted for Ce and Zn. The final composition of Example 19 on a wt% basis was 0.81 Pd, 0.70 Pb, 2.9 K. The final composition of Example 20 on a wt% basis was 0.80 Pd, 0.68 Sr, 2.7 K. For example 19 a 11.71g(13.2cc) charge of catalyst was placed in the reactor for testing. In example 20 a 10.95g(15.4cc) charge was used. As shown in Table 2, the lead promoted catalyst appeared to deactivate more rapidly than expected, while the strontium promoted catalyst was of low activity and poor selectivity. The pressure shown in this table is equivalent to 7.93 bar.

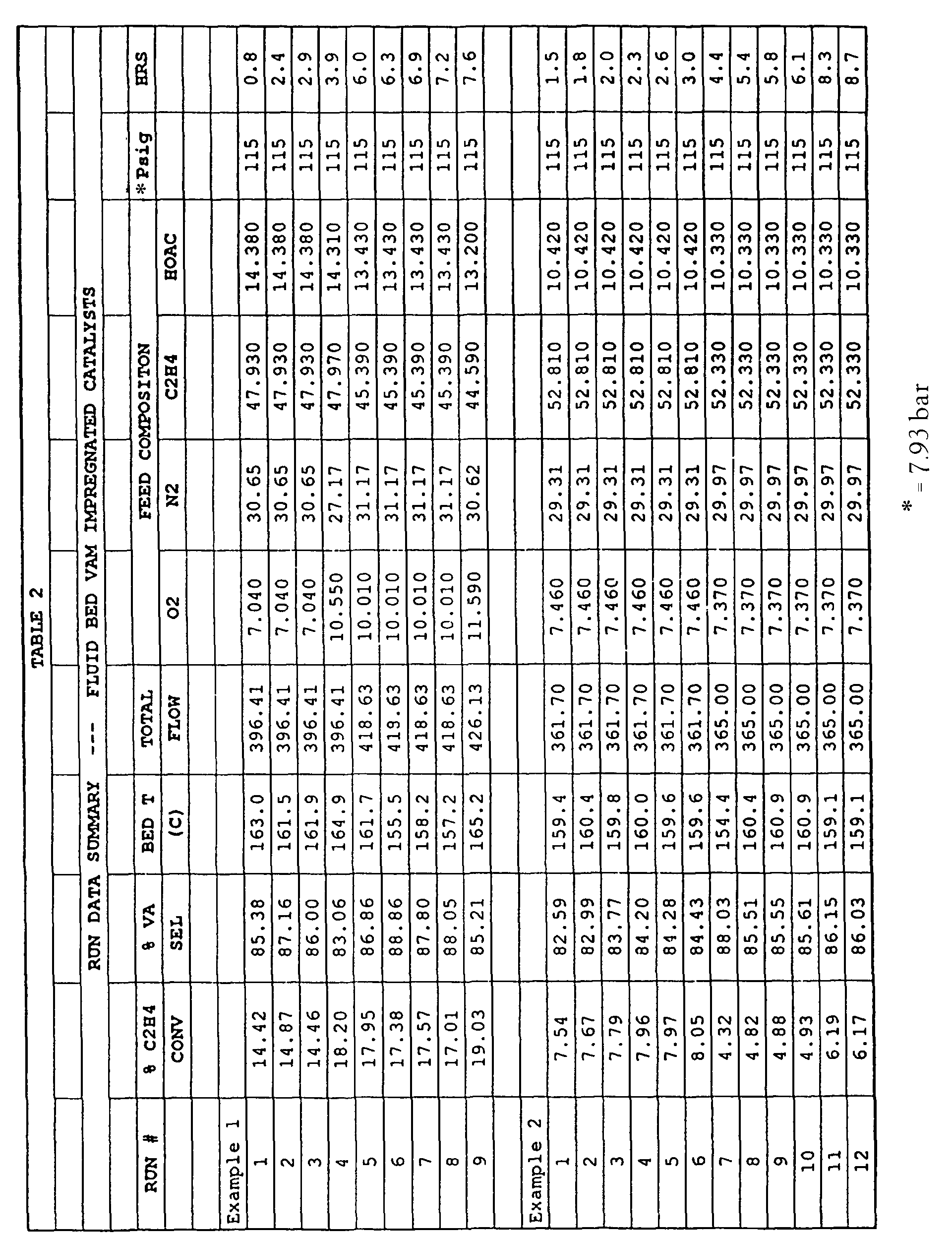

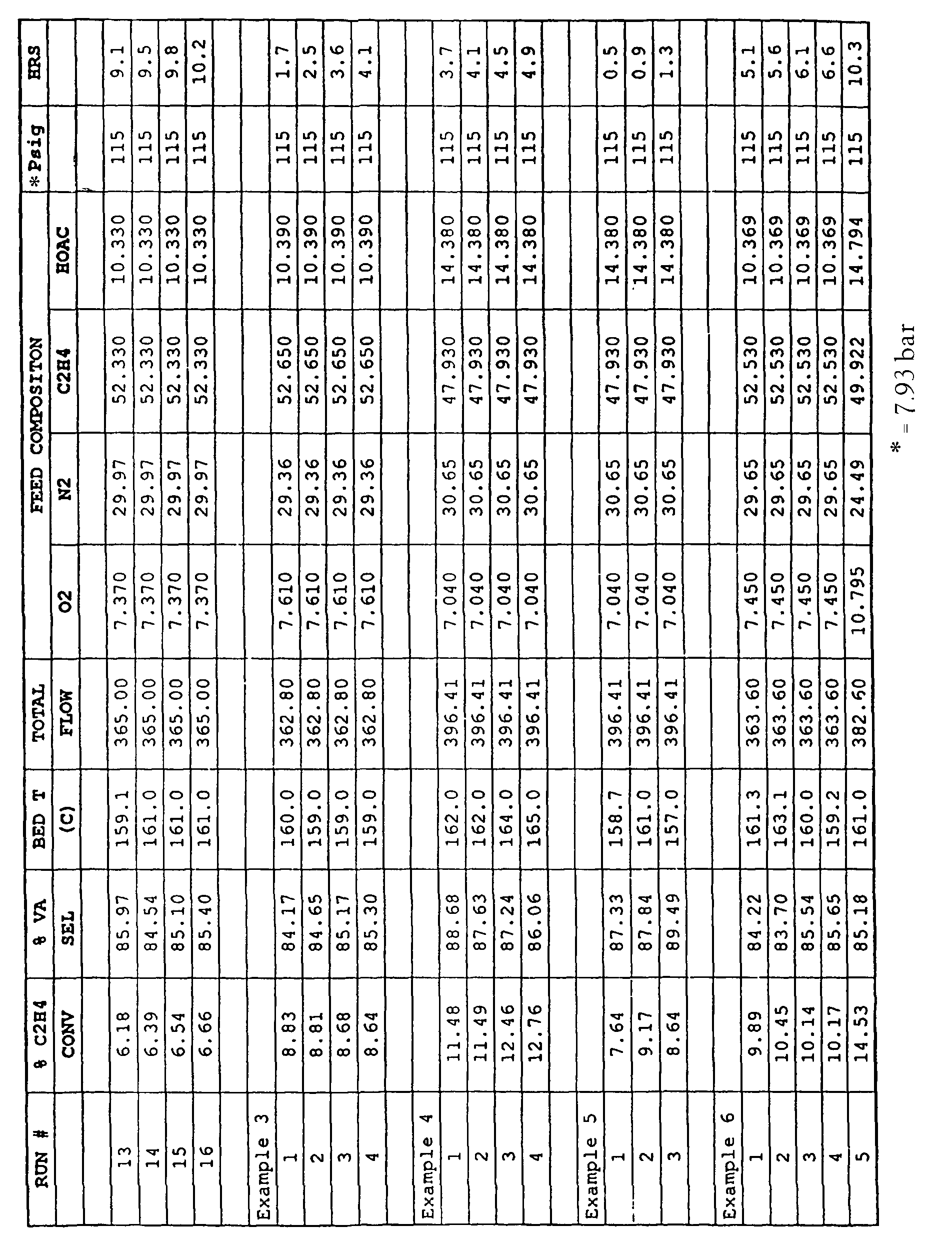

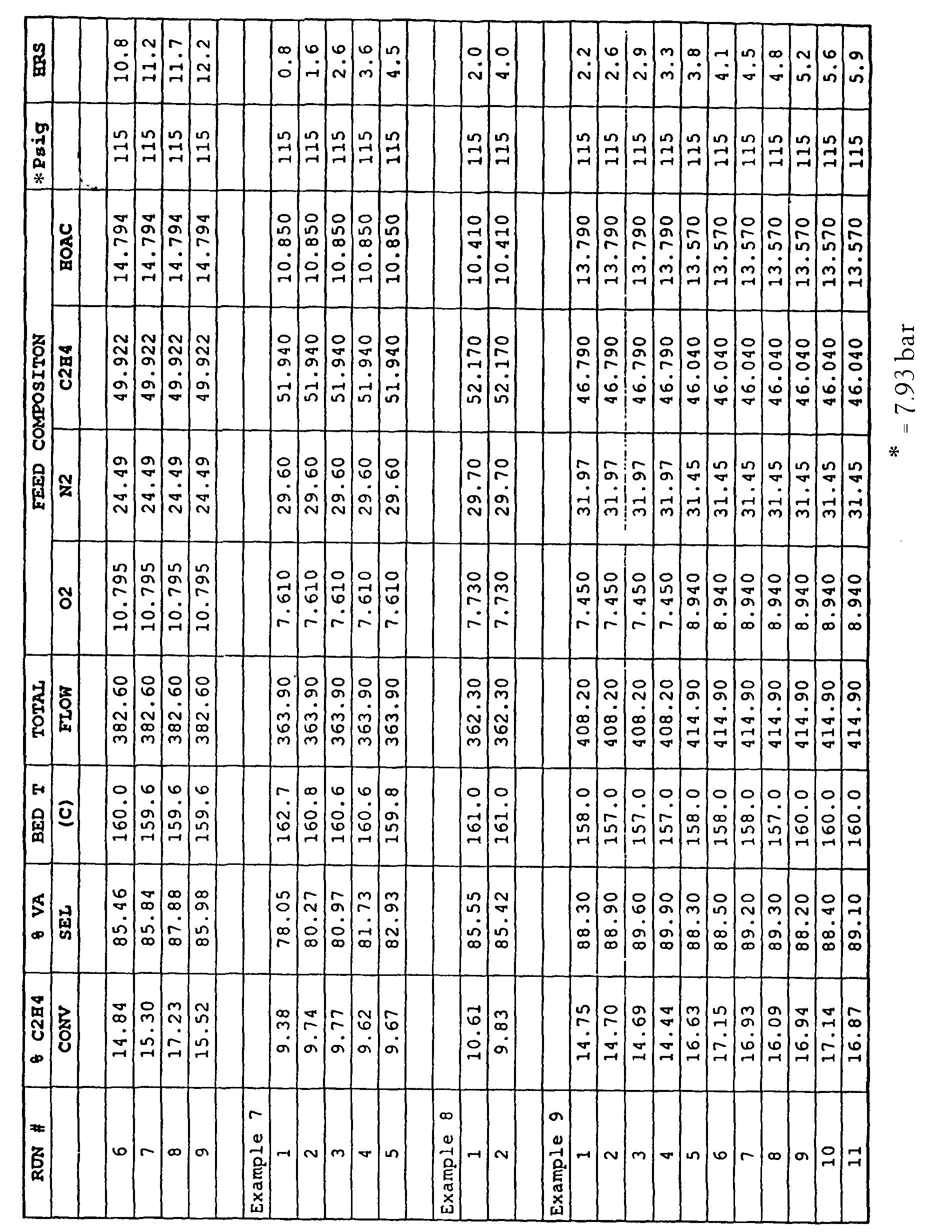

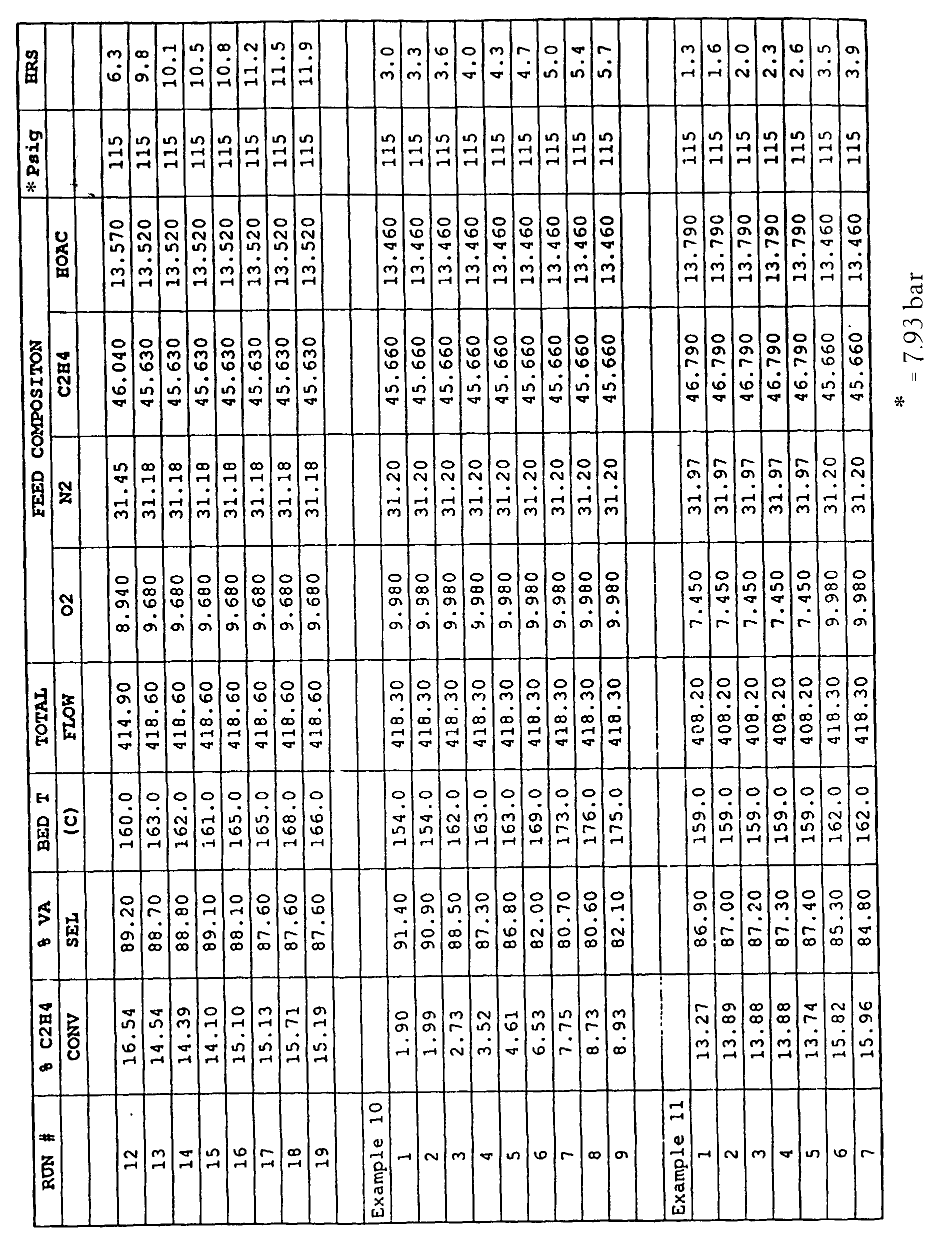

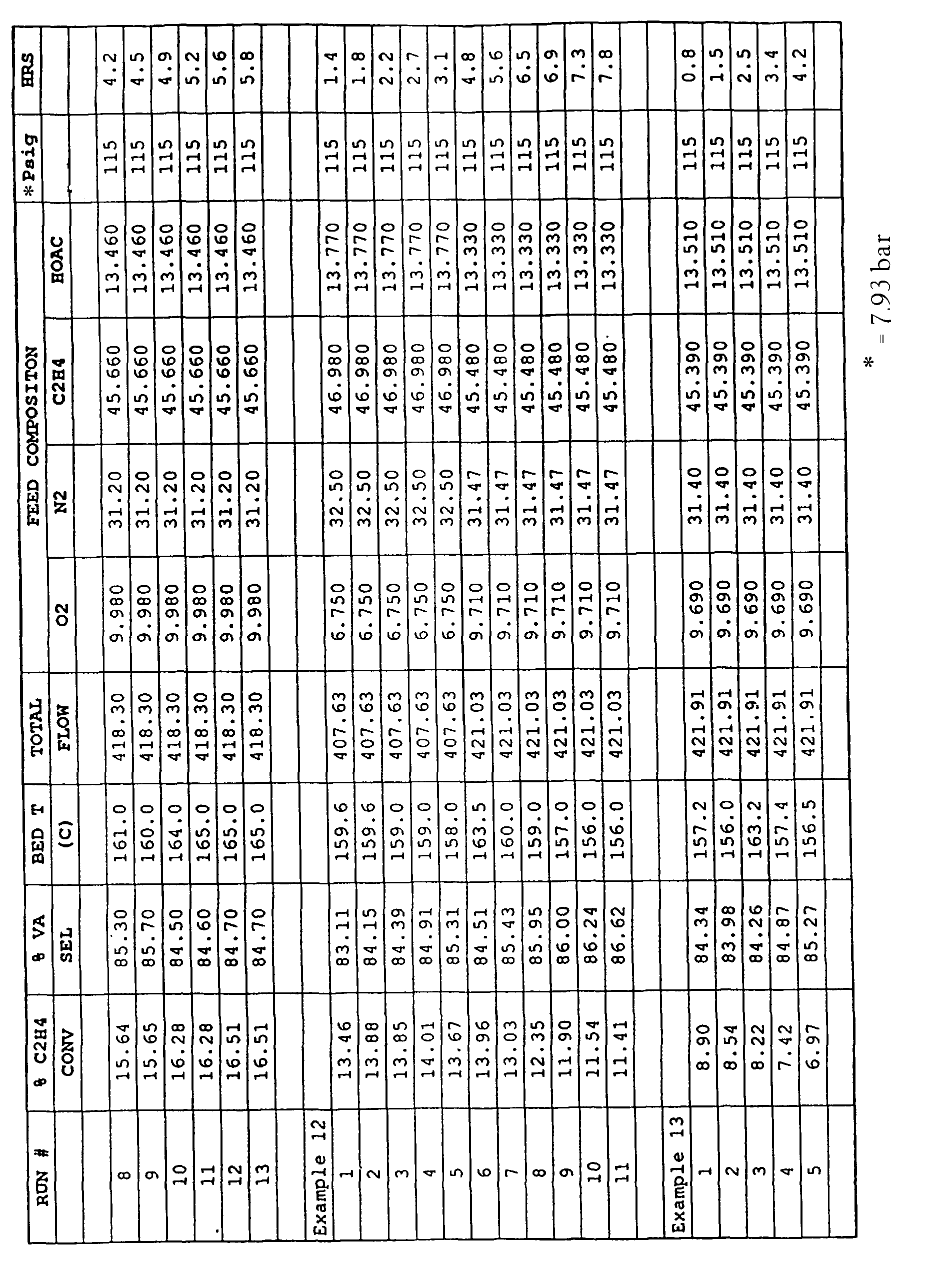

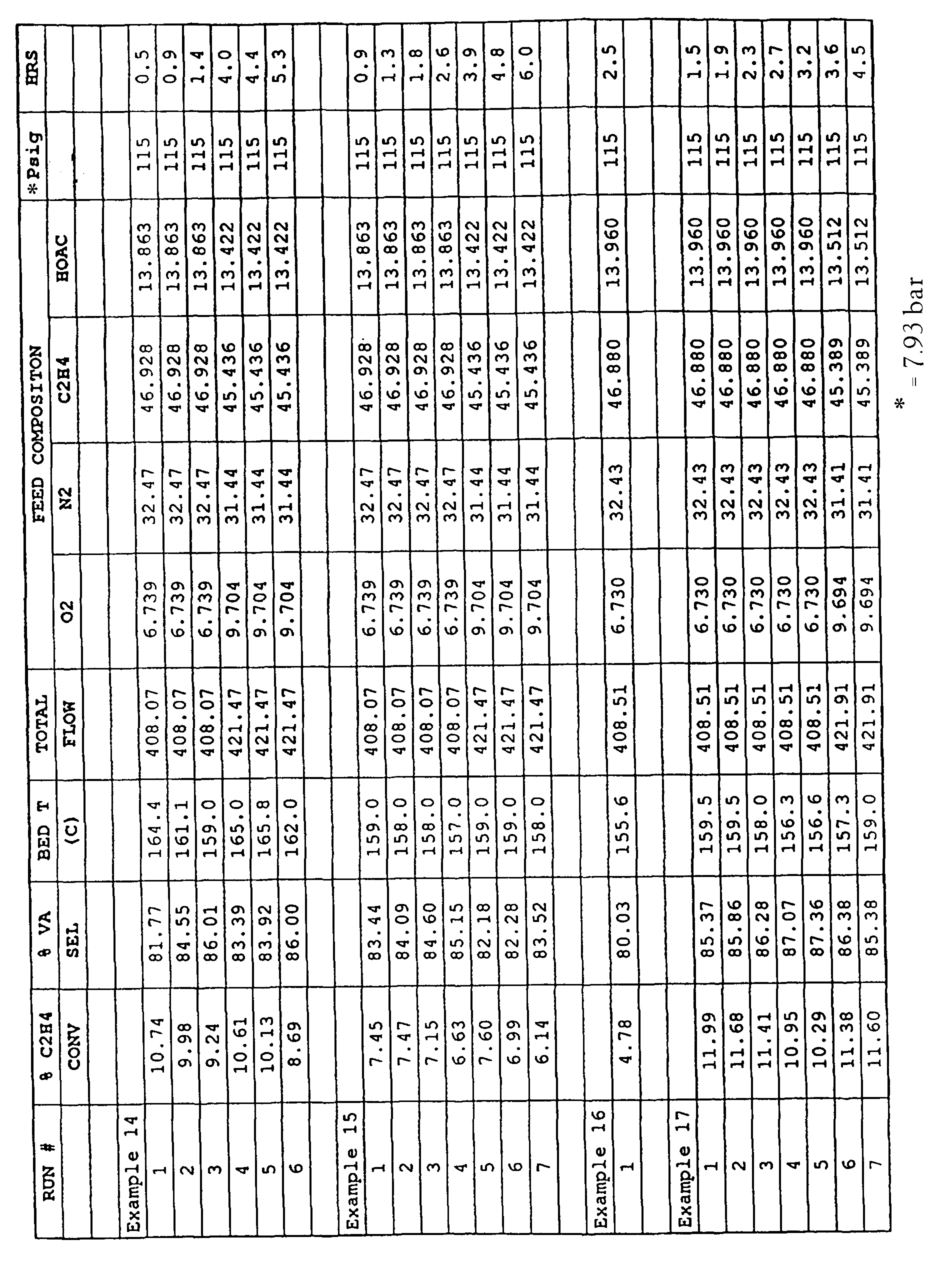

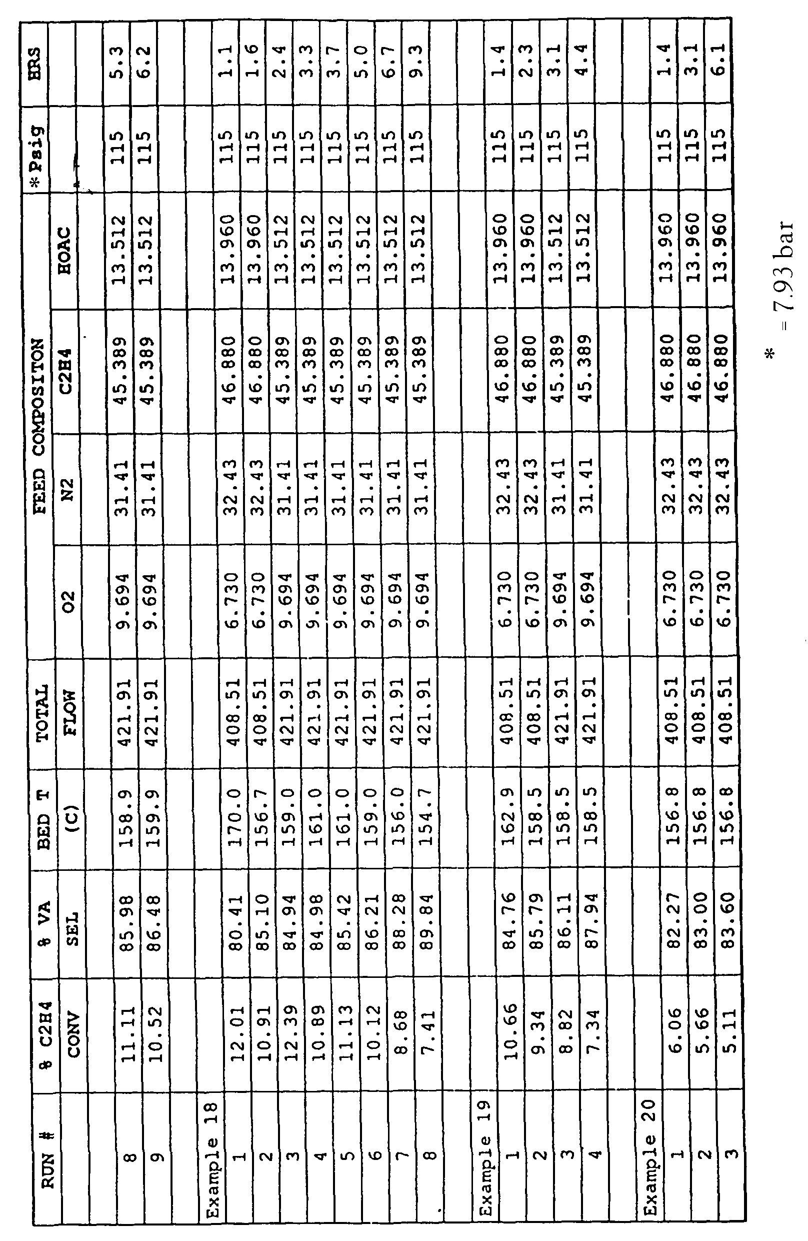

## Claims

1. A fluid bed vinyl acetate catalyst **characterised by** the following formula comprising Pd-M-A wherein M equals barium, gold, lanthanum, niobium, cerium, zinc, lead, calcium, strontium, antimony, or mixtures thereof; and A equals at least one alkali metal impregnated on a pre-formed substantially inert microspheroidal particulate support which support comprises a mixture of substantially inert microspheroidal particles having a pore volume of between 0.2 to 0.7 cc/g, a surface area of between 100 to 200 m²/g and at least 50% of said particles are less than 10⁻⁴ m (100 microns).

2. A catalyst as claimed in claim 1 wherein at least 75% of the microspheroidal support particles are below 10⁻⁴ m (100 microns).

3. A catalyst as claimed in claim 1 or claim 2 wherein the substantially inert microspheroidal particles are selected from the group consisting of silica, zirconia, alumina and mixtures thereof.

4. A catalyst as claimed in claim 3 wherein the substantially inert microspheroidal particles are selected to be silica.

5. A catalyst as claimed in any one of claims 1 to 4 wherein M is selected to be antimony.

6. A catalyst as claimed in any one of claims 1 to 4 wherein M is selected to be gold in the absence of barium.

7. A catalyst as claimed in claim 1 wherein the pore volume is between 0.3 and 0.65 cc/g, the surface area is between 100 to 195 m²/g and at least 75% of the particles are below 10⁻⁴ m (100 microns).

8. A catalyst as claimed in any one of the preceding claims in which the substantially inert support is made by a process comprising mixing 80 to 20 wt% of an aqueous sol comprising substantially inert microspheroidal particles with 20 to 80 wt% solid substantially inert particulate material to form an aqueous mixture, spray drying said aqueous mixture, and calcining said particles to form said substantially inert support.

9. A catalyst as claimed in claim 8 in which the aqueous sol comprises silica sol having a mean particle size of the silica of about 4 x 10⁻⁸ to 8 x 10⁻⁸ m (40 to 80 millimicrons).

10. A catalyst as claimed in claim 8 or claim 9 in which the solid substantially inert particulate material comprises silica having little or no micropores, mesopore volume of about 1.5 to 0.25 cc/g and a surface area of 80 to 200 m²/g.

11. A catalyst as claimed in any one of the preceding claims in which the weight percent of palladium is 0.1 to 5.0 wt%, preferably 0.2 to 4.0 wt% and most preferably 0.3 to about 1.0 wt%.

12. A catalyst as claimed in any one of the preceding claims comprising greater than 0 to 10 wt% alkali metal, preferably 0.1 to 8.0 wt% alkali metal, most preferably 0.1 to 5.0 wt% alkali metal.

13. A catalyst as claimed in any one of the preceding claims comprising greater than 0 to 5.0 wt% M, preferably 0.1 to 4.0 wt% M, most preferably 0.1 to 3.0 wt% M.

14. A process for the manufacture of a fluid bed vinyl acetate catalyst as claimed in any one of the preceding claims comprising impregnating a preformed microspheroidal substantially inert support wherein at least 50% of the particles have a size below 10⁻⁴ m (100 microns) with an aqueous solution substantially free of organic solvent comprising halide-free metal salt of Pd, M and at least one alkali metal and drying the impregnated microspheroidal support.

15. A process for manufacturing a fluid bed vinyl acetate catalyst as claimed in any one of claims 1 to 13 comprising impregnating a pre-formed substantially inert microspheroidal particulate support wherein at least 50% of the particles have a size below 10⁻⁴ m (100 microns) with a solution comprising a halide-free metal salt of the palladium, M, and at least one alkali metal and drying the impregnated preformed support.

16. A process as claimed in claim 14 or claim 15 wherein the alkali metal is separately impregnated onto the microspheroidal support material prior to drying the support.

17. A process as claimed in claim 16 wherein the alkali metal is impregnated onto the microspheroidal support subsequent to impregnating the support with the solution comprising the halide-free salts of palladium and M.

18. A process as claimed in any one of the claims 14 to 17 which further comprises drying the catalyst at a temperature up to about 80°C.

19. A process as claimed in any one of claims 14 to 18 wherein the solution comprising a halide-free metal salt of the palladium, M and at least one alkali metal is an aqueous solution substantially free of organic solvent.

20. A process for the production of vinyl acetate which comprises reacting ethylene, acetic acid and oxygen together in the gas phase in the presence of a fluidized bed of catalyst wherein the catalyst is a catalyst manufactured by a process as claimed in any one of claims 14 to 19 or is a catalyst as claimed in any one of claims 1 to 13.

## Patentansprüche

1. Fließbett-Vinylacetat-Katalysator, **gekennzeichnet durch** die folgende Formel, umfassend Pd-M-A, worin M Barium, Gold, Lanthan, Niob, Cer, Zink, Blei, Calcium, Strontium, Antimon oder Gemischen davon entspricht; und A mindestens einem Alkalimetall entspricht, das auf einem vorgeformten, im wesentlichen inerten, mikrosphäroidalen partikulären Träger imprägniert ist, wobei der Träger ein Gemisch aus im wesentlichen inerten mikrosphäroidalen Teilchen mit einem Porenvolumen zwischen 0,2 und 0,7 cm³/g, einer Oberfläche zwischen 100 und 200 m²/g umfaßt und mindestens 50 % der Teilchen weniger als 10⁻⁴ m (100 µm) betragen.

2. Katalysator nach Anspruch 1, wobei mindestens 75 % der mikrosphäroidalen Trägerteilchen unter 10⁻⁴ m (100 µm) liegen.

3. Katalysator nach Anspruch 1 oder Anspruch 2, wobei die im wesentlichen inerten mikrosphäroidalen Teilchen aus der Gruppe, bestehend aus Siliciumdioxid, Zirkoniumdioxid, Aluminiumoxid und Gemischen davon, ausgewählt sind.

4. Katalysator nach Anspruch 3, wobei die im wesentlichen inerten mikrosphäroidalen Teilchen Siliciumdioxid sind.

5. Katalysator nach einem der Ansprüche 1 bis 4, wobei M Antimon ist.

6. Katalysator nach einem der Ansprüche 1 bis 4, wobei M aus Gold in Abwesenheit von Barium ausgewählt ist.

7. Katalysator nach Anspruch 1, wobei das Porenvolumen zwischen 0,3 und 0,65 cm³/g, die Oberfläche zwischen 100 und 195 m²/g liegt und mindestens 75 % der Teilchen unter 10⁻⁴ m (100 µm) liegen.

8. Katalysator nach einem der vorhergehenden Ansprüche, wobei der im wesentlichen inerte Träger durch ein Verfahren hergestellt wird, umfassend das Mischen von 80 bis 20 Gew.-% eines wässerigen Sols, umfassend im wesentlichen inerte mikrosphäroidale Teilchen mit 20 bis 80 Gew.-% festes, im wesentlichen inertes partikuläres Material, um ein wässeriges Gemisch zu bilden, Sprühtrocknen des wässerigen Gemisches und Kalzinieren der Teilchen, um einen im wesentlichen inerten Träger zu bilden.

9. Katalysator nach Anspruch 8, wobei das wässerige Sol Siliciumdioxidsol mit einer mittleren Teilchengröße des Siliciumdioxids von 4 x 10⁻⁸ bis 8 x 10⁻⁸ m (40 bis 80 Millimikrometer) umfaßt.

10. Katalysator nach Anspruch 8 oder Anspruch 9, wobei das feste, im wesentlichen inerte partikuläre Material Siliciumdioxid mit wenig oder keinen Mikroporen, einem Mesoporenvolumen von etwa 1,5 bis 0,25 cm³/g und einer Oberfläche von 80 bis 200 m²/g umfaßt.

11. Katalysator nach einem der vorhergehenden Ansprüche, wobei das prozentuale Gewicht von Palladium 0,1 bis 5,0 Gew.-%, vorzugsweise 0,2 bis 4,0 Gew.-% und am stärksten bevorzugt 0,3 bis etwa 1,0 Gew.-% beträgt.

12. Katalysator nach einem der vorhergehenden Ansprüche, umfassend mehr als 0 bis 10 Gew.-% Alkalimetall, vorzugsweise 0,1 bis 8,0 Gew.-% Alkalimetall, am stärksten bevorzugt 0,1 bis 5,0 Gew.-% Alkalimetall.

13. Katalysator nach einem der vorhergehenden Ansprüche, umfassend mehr als 0 bis 5,0 Gew.-% M, vorzugsweise 0,1 bis 4,0 Gew.-% M, am stärksten bevorzugt 0,1 bis 3,0 Gew.-% M.

14. Verfahren zur Herstellung eines Fließbett-Vinylacetat-Katalysators nach einem der vorhergehenden Ansprüche, umfassend das Imprägnieren eines vorgeformten mikrosphäroidalen im wesentlichen inerten Trägers, wobei mindestens 50 % der Teilchen eine Größe unter 10⁻⁴ m (100 µm) aufweisen, mit einer wässerigen Lösung, die im wesentlichen frei von organischem Lösungsmittel ist, umfassend Halogenid-freies Metallsalz von Pd, M und mindestens ein Alkalimetall, und Trocknen des imprägnierten mikrosphäroidalen Trägers.

15. Verfahren zur Herstellung eines Fließbett-Vinylacetat-Katalysators nach einem der vorhergehenden Ansprüche 1 bis 13, umfassend das Imprägnieren eines vorgeformten im wesentlichen inerten mikrosphäroidalen partikulären Trägers, wobei mindestens 50 % der Teilchen eine Größe unter 10⁻⁴ m (100 µm) aufweisen, mit einer Lösung, umfassend Halogenid-freies Metallsalz von Palladium, M und mindestens ein Alkalimetall, und Trocknen des imprägnierten mikrosphäroidalen Trägers.

16. Verfahren nach Anspruch 14 oder Anspruch 15, wobei das Alkalimetall separat auf dem mikrosphäroidalen Trägermaterial vor dem Trocknen des Trägers imprägniert wird.

17. Verfahren nach Anspruch 16, wobei das Alkalimetall auf den mikrosphäroidalen Träger nach dem Imprägnieren des Trägers mit einer Lösung, umfassend die Halogenid-freien Salze von Palladium und M, imprägniert wird.

18. Verfahren nach einem der vorhergehenden Ansprüche 14 bis 17, das außerdem das Trocknen des Katalysators bei einer Temperatur von bis zu etwa 80 °C umfaßt.

19. Verfahren nach einem der Ansprüche 14 bis 18, wobei die Lösung, umfassend ein Halogenid-freies Metallsalz von Palladium, M und mindestens ein Alkalimetall, eine wässerige Lösung ist, die im wesentlichen frei von organischem Lösungsmittel ist.

20. Verfahren zur Herstellung von Vinylacetat, das das Umsetzen von Ethylen, Essigsäure und Sauerstoff zusammen in der Gasphase in Gegenwart eines Fließbettes des Katalysators umfaßt, wobei der Katalysator ein Katalysator ist, der durch ein Verfahren nach einem der Ansprüche 14 bis 19 hergestellt wurde, oder ein Katalysator nach einem der Ansprüche 1 bis 13 ist.

## Revendications

1. Catalyseur en lit fluidisé pour la préparation d'acétate de vinyle, **caractérisé par** la formule suivante comprenant Pd-M-A dans laquelle M signifie le baryum, l'or, le lanthane, le niobium, le cérium, le zinc, le plomb, le calcium, le strontium, l'antimoine ou des mélanges de ceux-ci et A signifie au moins un métal alcalin imprégné sur un support particulaire microsphéroïdal préfaçonné sensiblement inerte, ledit support comprenant un mélange de particules microsphéroïdales sensiblement inertes ayant un volume de pores compris entre 0,2 et 0,7 cm³/g, une surface spécifique comprise entre 100 et 200 m²/g, et au moins 50 % desdites particules ont une taille inférieure à 10⁻⁴ m (100 micromètres).

2. Catalyseur selon la revendication 1, dans lequel au moins 75 % des particules du support microsphéroïdal ont une taille inférieure à 10⁻⁴ m (100 micromètres).

3. Catalyseur selon la revendication 1 ou la revendication 2, dans lequel les particules microsphéroïdales sensiblement inertes sont choisies dans l'ensemble constitué de silice, d'oxyde de zirconium, d'alumine et de mélanges de ceux-ci.

4. Catalyseur selon la revendication 3, dans lequel les particules microsphéroïdales sensiblement inertes sont choisies de façon à être de la silice.

5. Catalyseur selon l'une quelconque des revendications 1 à 4, dans lequel M est choisi de façon à être de l'antimoine.

6. Catalyseur selon l'une quelconque des revendications 1 à 4, dans lequel M est choisi de façon à être de l'or en l'absence de baryum.

7. Catalyseur selon la revendication 1, dans lequel le volume de pores est compris entre 0,3 et 0,65 cm³/g, la surface spécifique est comprise entre 100 et 195 m²/g, et au moins 75 % des particules ont une taille inférieure à 10⁻⁴ m (100 micromètres).

8. Catalyseur selon l'une quelconque des revendications précédentes, dans lequel le support sensiblement inerte est préparé au moyen d'un procédé comprenant le mélange de 80 à 20 % en poids d'un sol aqueux comprenant des particules microsphéroïdales sensiblement inertes avec 20 à 80 % en poids d'une matière particulaire solide sensiblement inerte pour former un mélange aqueux, le séchage par pulvérisation dudit mélange aqueux et la calcination desdites particules pour former ledit support sensiblement inerte.

9. Catalyseur selon la revendication 8, dans lequel le sol aqueux comprend un sol de silice ayant une taille moyenne des particules de la silice de 4 x 10⁻⁸ à 8 x 10⁻⁸ m (40 à 80 millimicromètres).

10. Catalyseur selon la revendication 8 ou la revendication 9, dans lequel la matière particulaire solide sensiblement inerte comprend de la silice ayant peu ou pas de micropores, un volume de mésopores d'environ 1,5 à 0,25 cm³/g et une surface spécifique de 80 à 200 m²/g.

11. Catalyseur selon l'une quelconque des revendications précédentes, dans lequel le pourcentage en poids de palladium est de 0,1 à 5,0 % en poids, de préférence de 0,2 à 4,0 % en poids et de la façon la plus préférée de 0,3 à environ 1,0 % en poids.

12. Catalyseur selon l'une quelconque des revendications précédentes, comprenant plus de 0 à 10 % en poids d'un métal alcalin, de préférence de 0,1 à 8,0 % en poids d'un métal alcalin, de la façon la plus préférée de 0,1 à 5,0 % en poids d'un métal alcalin.

13. Catalyseur selon l'une quelconque des revendications précédentes, comprenant plus de 0 à 5,0 % en poids de M, de préférence de 0,1 à 4,0 % en poids de M, de la façon la plus préférée de 0,1 à 3,0 % en poids de M.

14. Procédé de fabrication d'un catalyseur en lit fluidisé pour la préparation d'acétate de vinyle selon l'une quelconque des revendications précédentes, comprenant l'imprégnation d'un support microsphéroïdal préfaçonné sensiblement inerte, dans lequel au moins 50 % des particules ont une taille inférieure à 10⁻⁴ m (100 micromètres), avec une solution aqueuse sensiblement exempte de solvant organique, comprenant un sel métallique de Pd exempt d'halogénure, M et au moins un métal alcalin et le séchage du support microsphéroidal imprégné.

15. Procédé de fabrication d'un catalyseur en lit fluidisé pour la préparation d'acétate de vinyle selon l'une quelconque des revendications 1 à 13, comprenant l'imprégnation d'un support particulaire microsphéroïdal préfaçonné sensiblement inerte, dans lequel au moins 50 % des particules ont une taille inférieure à 10⁻⁴ m (100 micromètres), avec une solution comprenant un sel métallique de palladium exempt d'halogénure, M et au moins un métal alcalin et le séchage du support imprégné préfaçonné.

16. Procédé selon la revendication 14 ou la revendication 15, dans lequel le métal alcalin est imprégné séparément sur la matière formant le support microsphéroïdal avant le séchage du support.

17. Procédé selon la revendication 16, dans lequel le métal alcalin est imprégné sur le support microsphéroïdal après l'imprégnation du support avec la solution comprenant les sels de palladium exempts d'halogénure et M.

18. Procédé selon l'une quelconque des revendications 14 à 17, comprenant en outre le séchage du catalyseur à une température allant jusqu'à environ 80°C.

19. Procédé selon l'une quelconque des revendications 14 à 18, dans lequel la solution comprenant un sel métallique de palladium exempt d'halogénure, M et au moins un métal alcalin est une solution aqueuse sensiblement exempte de solvant organique.

20. Procédé de préparation d'acétate de vinyle, comprenant la mise en réaction d'éthylène, d'acide acétique et d'oxygène ensemble en phase gazeuse en présence d'un lit fluidisé de catalyseur, dans lequel le catalyseur est un catalyseur fabriqué au moyen d'un procédé selon l'une quelconque des revendications 14 à 19 ou est un catalyseur selon l'une quelconque des revendications 1 à 13.
